(19) Europäisches Patentamt
European Patent Office
Office européen des brevets

(11) EP 3 949 944 A1

(12) **EUROPEAN PATENT APPLICATION**
published in accordance with Art. 153(4) EPC

(43) Date of publication:
**09.02.2022 Bulletin 2022/06**

(21) Application number: **20784008.3**

(22) Date of filing: **06.04.2020**

(51) International Patent Classification (IPC):
***A61K 8/898*** *(2006.01)* ***A61K 8/19*** *(2006.01)*
***A61K 8/25*** *(2006.01)* ***A61K 8/29*** *(2006.01)*
***A61K 8/34*** *(2006.01)* ***A61K 8/42*** *(2006.01)*
***A61K 8/49*** *(2006.01)* ***A61K 8/58*** *(2006.01)*
***A61K 8/89*** *(2006.01)* ***A61Q 5/00*** *(2006.01)*
***A61Q 5/06*** *(2006.01)*

(52) Cooperative Patent Classification (CPC):
**A61K 8/19; A61K 8/25; A61K 8/29; A61K 8/34; A61K 8/42; A61K 8/49; A61K 8/58; A61K 8/89; A61K 8/898; A61Q 5/00; A61Q 5/06**

(86) International application number:
**PCT/JP2020/015484**

(87) International publication number:
**WO 2020/204205 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.04.2019 JP 2019072119**
**25.12.2019 JP 2019234992**

(71) Applicant: **Kao Corporation**
**Chuo-ku**
**Tokyo 103-8210 (JP)**

(72) Inventors:
- **SAITO, Yoichi**
**Tokyo 131-8501 (JP)**
- **NAKAOKA, Shiho**
**Tokyo 131-8501 (JP)**

(74) Representative: **Hoffmann Eitle**
**Patent- und Rechtsanwälte PartmbB**
**Arabellastraße 30**
**81925 München (DE)**

(54) **HAIR COSMETIC**

(57) The present invention relates to a hair cosmetic containing the following components (A) to (D):
(A) an interference pearl pigment,
(B) other pigment than the component (A),
(C) a film-forming polymer, and
(D) a nonaqueous solvent,
wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water is 40% by mass or less.

EP 3 949 944 A1

## Description

Field of the Invention

The present invention relates to a hair cosmetic.

Background of the Invention

A hair dye is classified into a permanent hair dye in which an oxidative dye is used for color development to produce a color in hair (e.g., a hair coloring), a semi-permanent hair dye in which a direct dye is subjected to penetration and absorption on the hair to produce a color (e.g., a hair manicure), a temporary hair dye in which a colored film containing a colorant (mainly a pigment) is formed on the hair to produce a color (e.g., a hair mascara), and so on. Of these, the temporary hair dye is preferred by consumers as a hair coloring cosmetic that can be freely enjoyed because it has less damage to the hair, good removability by shampooing, and convenience of use. As a conventional temporary hair dye, a temporary hair dye composition containing a film-forming polymer is known in order to fix a colorant as a colored film onto the hair, impart water resistance to this colored film, and prevent color transfer to the skin or clothing.

Among the temporary hair dyes, there are those for the purpose of dyeing the gray hair and those for imparting a brilliant color to the hair for the purpose of improving fashion ability.

Regarding the latter, temporary hair dyes for imparting a brilliant color to the black hair are being studied. For example, PTL 1 discloses a composition containing a pigment and two kinds of organopolysiloxanes in a predetermined ratio as a temporary hair dye composition in which styling properties are not impaired even after coloring the hair, combability is good, color fading during rubbing and peeling of colored film are not accompanied, excellent abrasion resistance is obtained, and further, excellent rinsability is provided even when a sufficient amount of the hair dye is applied in order to impart a brilliant color to the black hair.

PTLs 2 and 3 disclose a composition containing a non-crosslinked anionic copolymer containing a predetermined structural unit and a polyether-modified silicone in a predetermined ratio and also containing a pigment and also a composition containing two types of copolymers containing a predetermined structural unit and a pigment as a temporary hair dye composition that is neither sticky nor stiff even after coloring the hair and excellent in combability, abrasion resistance, water resistance, and rinsability.

Citation List

Patent Literature

PTL 1: JP 2017-114815 A
PTL 2: JP 2019-6690 A
PTL 3: JP 2019-6691 A

Summary of the Invention

The present invention relates to the following.

[1] A hair cosmetic containing the following components (A) to (D):

(A) an interference pearl pigment,
(B) other pigment than the component (A),
(C) a film-forming polymer, and
(D) a nonaqueous solvent,

wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water is 40% by mass or less.

[2] Use of a composition as a hair cosmetic, the composition containing the following components (A) to (D):

(A) an interference pearl pigment,

(B) other pigment than the component (A),
(C) a film-forming polymer, and
(D) a nonaqueous solvent,

wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water is 40% by mass or less.

Detailed Description of the Invention

[Hair Cosmetic]

The hair cosmetic of the present invention contains the following components (A) to (D):

(A) an interference pearl pigment,
(B) other pigment than the component (A),
(C) a film-forming polymer, and
(D) a nonaqueous solvent,

wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water is 40% by mass or less.

In view of the fact that the hair cosmetic of the present invention has the aforementioned constitution, drying after application is fast, and when used as a temporary hair dye, color transfer to the skin or clothing after hair dyeing is less, a feeling on the hair is favorable, and color-developing behavior is hardly influenced by a base hair color, so that a fixed hair dyeing effect can be imparted. With respect to the wording "color-developing behavior is hardly influenced by a base hair color, so that a fixed hair dyeing effect is imparted" as referred to herein, in detail, it is meant that regardless of whether the base hair color is a bright color or a dark color, a color change is produced according to the applied color without significantly changing the brightness (lightness) of the hair before and after hair dyeing.

Although the temporary hair dye is used in various use modes, a type in which it is applied using an applying tool, such as a brush, a comb, and a sponge is suitable. In the temporary hair dye of such a type, it is required to be rapidly dried after application on the hair such that the colorant does not attach to a hand or the like. In addition, as for the temporary hair dye, it is also important that color transfer to the skin or clothing after hair dyeing is less, and stiffness of the hair after hair dyeing is not produced.

Furthermore, in the case of a temporary hair dye for the purpose of improving the fashionability, though bright color development is preferred, the color development of a conventional temporary hair dye is readily influenced by the base hair color, and when using a temporary hair dye having the same composition, a degree of color development was different depending upon whether the hair with a dark color, such as black hair, or the hair with a bright color, such as bleached hair, is to be dyed. For example, if it is intended to dye the hair with a dark color into a bright color, a color change is hardly seen, whereas in a hair dye so as to provide a bright color development in the hair with a dark color, it was involved such a problem that when dyeing the hair with a bright color, the color development is excessively gaudy.

The present invention relates to a hair cosmetic which is suitable as a temporary hair dye and fast in drying after application and in which color transfer to the skin or clothing after hair dyeing is less, a feeling on the hair is favorable, and color-developing behavior is hardly influenced by a base hair color, so that a fixed hair dyeing effect can be imparted.

The present inventors have found that a hair cosmetic not only containing an interference pearl pigment and other pigment in predetermined amounts and ratio but also containing a film-forming polymer and a nonaqueous solvent, wherein the content of water is not more than a predetermined amount, satisfies the aforementioned requirements.

In accordance with the present invention, it is possible to provide a hair cosmetic which is suitable as a temporary hair dye and fast in drying after application and in which color transfer to the skin or clothing after hair dyeing is less, a feeling on the hair is favorable, and color-developing behavior is hardly influenced by a base hair color, so that a fixed hair dyeing effect can be imparted.

In the present invention, though the reason why the aforementioned effects are obtained is not always elucidated yet, it may be considered as follows.

In hair dyes, such as a temporary hair dye, a pigment is generally used as a colorant. However, for example, in a temporary hair dye using only an organic pigment as the colorant, when dyeing the hair with a dark color, such as black hair, since a color change is hardly seen, in order to provide sufficient color development, it was needed to blend

an organic pigment in a considerable amount. On the other hand, in a temporary hair dye having a considerable amount of an organic pigment blended therein, when applied on the hair with a bright color, such as bleached hair, the color development was too gaudy, so that such was not practically useful.

With respect to the aforementioned problem that the degree of color development of the temporary hair dye is readily influenced by the base hair color, the present inventors made extensive and intensive investigations. As a result, it has been found that the aforementioned problem can be solved by using, as the pigment component, an interference pearl pigment that is the component (A) and other pigment than the component (A) (component (B)) in predetermined amounts and ratio.

The interference pearl pigment that is the component (A) is a pigment exhibiting a pearly luster owing to a reflection and interference action of light and refers to one having a coating layer on a surface of a core particle, the coating layer including a reflection and interference layer and having translucency. The core particle is typically a semi-transparent tabular particle, and the reflection and interference layer is constituted of a colorless metal oxide, such as titanium dioxide, or a colorless metal. In addition, though the coating layer occasionally exhibits a wavelength color originated from its reflected light depending upon the thickness of the reflection and interference layer included therein, the coating layer per se has translucency.

In consequence, even when the component (A) is singly applied on the hair with a bright color, the color change is hardly seen. However, when it is applied on the hair with a dark color, not only a glittering luster is provided owing to a reflection and interference action of light, but also a color change is readily seen. Accordingly, when using the component (A), even if a large amount of a colorant that is the component (B), such as an organic pigment, is not blended, it may be considered that when applying on the hair with a dark color, a hair cosmetic exhibiting sufficient color development is obtained. In addition, it may be considered that when applying the foregoing hair cosmetic on the hair with a bright color, desired color development can be imparted without significantly changing the brightness of hair.

In the hair cosmetic of the present invention, the total content of the component (A) and the component (B) is 2.0% by mass or more and 12.0% by mass or less, the mass ratio [(A)/{(A) + (B)}] of the component (A) to the total content of the component (A) and the component (B) is 0.30 or more and 0.96 or less, and the mass ratio [(C)/{(A) + (B)}] of the component (C) to the total content of the component (A) and the component (B) is 0.5 or more and 2.4 or less. When regulating the hair cosmetic of the present invention to the foregoing ranges, it may be considered that in the case where the hair cosmetic is a temporary hair dye, the color-developing behavior is hardly influenced by the base hair color, so that a fixed hair dyeing effect can be imparted.

In view of the fact that the hair cosmetic of the present invention contains the component (C), it is able to impart abrasion resistance, water resistance, removability by shampooing, softness, high color developability, and so on, and on the occasion when it is used as a temporary hair dye, color transfer to the skin or clothing after hair dyeing is less, and a feeling on the hair is also favorable. Furthermore, in view of the fact that not only the hair cosmetic of the present invention further contains a non-aqueous solvent as the component (D), but also the content of water is 40% by mass or less, drying after applying on the hair is fast, and handling properties during use are favorable.

Examples of the hair cosmetic include a hair shampoo, a hair rinse, a hair conditioning agent, a hair treatment agent (inclusive of a non-rinsing-off type), a hair styling agent, a hair dye, and a hair growth promoter. Of these, the hair cosmetic of the present invention is preferably a hair dye, and more preferably a temporary hair dye from the viewpoint of usefulness of the effects of the present invention.

<Component (A): Interference Pearl Pigment>

The hair cosmetic of the present invention contains an interference pearl pigment as the component (A). In view of the fact that the hair cosmetic of the present invention contains the component (A), in the case where the hair cosmetic is a temporary hair dye, the color-developing behavior is hardly influenced by a base hair color owing to the aforementioned function mechanism, so that a fixed hair dyeing effect can be imparted.

As mentioned above, the interference pearl pigment is a pigment exhibiting a pearly luster owing to a reflection and interference action of light and having a coating layer on a surface of a core particle, the coating layer including a reflection and interference layer and having translucency. In the present invention, the coating layer in the interference pearl pigment does not substantially contain a colored metal oxide and a colored metal. It is meant by the wording "does not substantially contain" that the coating layer does not contain any of a colored metal oxide and a colored metal within a range where the translucency of the coating layer per se does not appear. The content of the colored metal oxide and the colored metal in the coating layer is preferably 3% by mass or less, more preferably 1% by mass or less, and still more preferably 0.1% by mass or less. Examples of the colored metal oxide include iron oxide, copper oxide, cobalt oxide, chromium oxide, and nickel oxide, and examples of the colored metal include gold and copper.

In the present invention, the interference pearl pigment is distinguished from a colored pearl pigment. The colored peal pigment is a pigment exhibiting a pearly luster owing to a reflection and interference action of light and has a coating layer on a surface of a core particle, and the foregoing coating layer is colored with the aforementioned colored

metal oxide or colored metal and does not have translucency. In consequence, the colored pearl pigment is different from the interference pearl pigment and has concealability.

A pigment having a coating layer on a surface of a core particle, the coating layer including a reflection and interference layer constituted of a colorless metal oxide or a colorless metal and a colored layer formed on a surface of the reflection and interference layer and constituted of an organic pigment is, in general, occasionally named as a composite pearl pigment. However, in the present invention, such a pigment is included in the interference pearl pigment so long as the coating layer has translucency as a whole.

Examples of a material of the core particle constituting the component (A) include mica, sericite, talc, kaolin, a smectite group clay mineral, bismuth oxychloride, synthetic mica, synthetic sericite, tabular silica, and tabular alumina. Of these, mica is preferred.

The reflection and interference layer included in the coating layer of the component (A) is preferably a layer formed of a thin film composed mainly of a colorless metal oxide, such as titanium dioxide, zinc oxide, and aluminum oxide, or a colorless metal, such as titanium, zirconium, zinc, tin, silicon, and aluminum, and it may be of a single-layered structure or a multi-layered structure. The colorless metal oxide or colorless metal constituting the reflection and interference layer is more preferably at least one selected from the group consisting of titanium and titanium dioxide, and still more preferably titanium dioxide.

The interference pearl pigment that is the component (A) is more preferably titanium dioxide-coated mica (micaceous titanium) from the viewpoint of imparting a fixed hair dyeing effect regardless of the influence by a base hair color in the case where the hair cosmetic is a temporary hair dye.

Although a ratio between the core particle and the reflection and interference layer constituting the component (A) is not particularly restricted, from the viewpoint of imparting a fixed hair dyeing effect regardless of the influence by a base hair color in the case where the hair cosmetic is a temporary hair dye, a mass ratio of the core particle to the colorless metal oxide and the colorless metal constituting the reflection and interference layer [(core particle)/(colorless metal oxide and colorless metal constituting the reflection and interference layer)] is preferably 25/75 to 80/20, more preferably 30/70 to 70/30, and still more preferably 50/50 to 65/35.

An average particle diameter of the component (A) is preferably 2 pm or more, more preferably 5 pm or more, and still more preferably 10 pm or more, and preferably 40 pm or less, more preferably 35 pm or less, and still more preferably 30 pm or less in terms of a volume median particle diameter (D50). A specific range of the volume median particle diameter (D50) of the component (A) is preferably 2 to 40 pm, more preferably 5 to 35 pm, and still more preferably 10 to 30 $\mu$m. When the D50 of the component (A) falls within the aforementioned range, during using the hair cosmetic as a temporary hair dye, a fixed hair dyeing effect can be imparted regardless of the influence by a base hair color.

The D50 of the component (A) is a value measured with a laser diffraction particle size analyzer.

Examples of a commercially available interference pearl pigment which can be used as the component (A) include "Timiron Super Blue" (titanium mica, mica: 51% by mass, titanium dioxide: 49% by mass, D50: 18 to 25 pm), "Timiron Super Gold" (titanium mica, mica: 60% by mass, titanium dioxide: 40% by mass, D50: 18 to 25 pm), "Timiron Super Green" (titanium mica, mica: 55% by mass, titanium dioxide: 45% by mass, D50: 18 to 25 pm), and "Timiron Super Red" (titanium mica, mica: 52% by mass, titanium dioxide: 48% by mass, D50: 18 to 25 pm), all being available from Merck.

The content of the component (A) in the hair cosmetic of the present invention is preferably 1.5% by mass or more, more preferably 2.5% by mass or more, still more preferably 3.0% by mass or more, yet still more preferably 3.5% by mass or more, and even yet still more preferably 4.0% by mass or more, and preferably 10% by mass or less, more preferably 9.5% by mass or less, still more preferably 9.0% by mass or less, and yet still more preferably 8.5% by mass or less from the viewpoint of imparting a fixed hair dyeing effect regardless of the influence by a base hair color in the case where the hair cosmetic is a temporary hair dye. A specific range of the content of the component (A) in the hair cosmetic of the present invention is preferably 1.5 to 10% by mass, more preferably 2.5 to 10% by mass, still more preferably 3.0 to 9.5% by mass, yet still more preferably 3.5 to 9.0% by mass, and even yet still more preferably 4.0 to 8.5% by mass.

<Component (B): Other Pigment than Component (A)>

The hair cosmetic of the present invention contains, as the component (B), other pigment than the component (A). In view of the fact that the hair cosmetic of the present invention contains the component (B), in the case where the hair cosmetic is a temporary hair dye, a desired color shade can be imparted, and by further jointly using the component (A), the color-developing behavior is hardly influenced by a base hair color, so that a fixed hair dyeing effect can be imparted.

As for the component (B), any pigment can be used irrespective of the shape (e.g., a spherical shape, an acicular shape, and a tabular shape) and the particle diameter, and the particle structure (e.g., a porous structure and a nonporous structure), and examples thereof include an inorganic pigment and an organic pigment other than the

component (A).

Examples of the inorganic pigment other than the component (A) include inorganic black pigments, such as carbon black, black iron oxide, and black titanium oxide; inorganic red pigments, such as iron oxide (red iron oxide), iron hydroxide, and iron titanate; inorganic brown pigments, such as y-iron oxide; inorganic yellow pigments, such as yellow iron oxide and ocher; inorganic blue pigments, such as ultramarine blue and Prussian blue; inorganic violet pigments, such as manganese violet and cobalt violet; inorganic green pigments, such as chromium oxide, chromium hydroxide, and cobalt titanate; inorganic white pigments, such as titanium oxide, zinc oxide, cerium oxide, and barium sulfate; metal powder pigments, such as gold powder, silver powder, copper powder, aluminum powder, and brass powder; and colored pearl pigments, such as iron oxide-coated mica, iron oxide-coated micaceous titanium, black iron oxide-coated mica, black iron oxide-coated micaceous titanium, yellow iron oxide-coated mica, iron oxide/black iron oxide-coated micaceous titanium, iron oxide/Prussian blue-coated micaceous titanium, iron oxide-coated bismuth oxychloride, and iron oxide-coated bismuth oxychloride mica.

Examples of the organic pigment include Red No. 201, Red No. 202, Red No. 203, Red No. 204, Red No. 205, Red No. 206, Red No. 207, Red No. 208, Red No. 219, Red No. 220, Red No. 221, Red No. 226, Red No. 228, Red No. 404, Red No. 405, Orange No. 203, Orange No. 204, Orange No. 401, Yellow No. 205, Yellow No. 401, and Blue No. 404.

Examples of the component (B) other than those mentioned above include glittering powders, such as a polyethylene terephthalate/aluminum/epoxy laminate and a polyethylene terephthalate/polyolefin laminated film.

The aforementioned pigments can be used alone or in appropriate combination of two or more thereof according to the desired color shape.

From the viewpoint of imparting a fixed hair dyeing effect regardless of the influence by a base hair color in the case where the hair cosmetic is a temporary hair dye, in the hair cosmetic of the present invention, an absolute value of a hue difference ΔH° measured by the following method (I) is preferably 30 or less, more preferably 15 or less, and still more preferably 10 or less. By selecting the absolute value of the hue difference ΔH° so as to fall within the aforementioned range, the effect for imparting a fixed hair dyeing effect regardless of the influence by a base hair color becomes high.

Method (I): With respect to the hair cosmetic, a sample A containing only the component (A) as the pigment (a sample in which the component (B) is not blended in the hair cosmetic, and the component (B) is replaced by the same amount of ethanol) and a sample B containing only the component (B) as the pigment (a sample in which the component (A) is not blended in the hair cosmetic, and the component (A) is replaced by the same amount of ethanol) are prepared. 2 g of the sample A is uniformly applied on a black plate of 2 cm × 2 cm and 2 g of the sample B is uniformly applied on a white plate of 2 cm × 2 cm, respectively and allowed to stand for natural drying at 25°C for 1 hour. A hue of the sample-applied surface of each of the plates is measured with a color-difference meter, and the absolute value of the hue difference ΔH° is determined from a difference therebetween.

The absolute value of the aforementioned hue difference ΔH° can be, for example, measured with a color-difference meter (CR-400, available from Konica Minolta, Inc.) by the method described in the section of Examples.

Although the content of the component (B) in the hair cosmetic of the present invention is not particularly restricted so long as the total content of the component (A) and the component (B) as mentioned later and also the mass ratio [(A)/{(A) + (B)}] of the component (A) to the total content of the component (A) and the component (B) fall within predetermined ranges, it is preferably 0.5% by mass or more, more preferably 0.8% by mass or more, and still more preferably 1.0% by mass or more from the viewpoint of imparting a desired color shade in the case where the hair cosmetic is a temporary hair dye. In addition, it is preferably 8.0% by mass or less, more preferably 7.0% by mass or less, still more preferably 6.0% by mass or less, yet still more preferably 5.0% by mass or less, and even yet still more preferably 4.0% by mass or less from the viewpoint of imparting a fixed hair dyeing effect regardless of the influence by a base hair color during using as a temporary hair dye. A specific range of the content of the component (B) in the hair cosmetic of the present invention is preferably 0.5 to 8.0% by mass, more preferably 0.5 to 7.0% by mass, still more preferably 0.8 to 6.0% by mass, yet still more preferably 0.8 to 5.0% by mass, and even yet still more preferably 1.0 to 4.0% by mass.

From the viewpoint of improving the hair dyeing effect on the hair with a dark color, such as black hair, during using as a temporary hair dye, the total content of the component (A) and the component (B) in the hair cosmetic of the present invention is 2.0% by mass or more, preferably 3.0% by mass or more, more preferably 3.5% by mass or more, still more preferably 4.0% by mass or more, yet still more preferably 5.0% by mass or more, and even yet still more preferably 6.0% by mass or more. In addition, the foregoing total content is 12.0% by mass or less, preferably 11.0% by mass or less, more preferably 10.0% by mass or less, and still more preferably 9.5% by mass or less. A specific range of the total content of the component (A) and the component (B) in the hair cosmetic of the present invention is 2.0 to 12.0% by mass, preferably 3.0 to 11.0% by mass, more preferably 3.5 to 11.0% by mass, still more preferably 4.0 to 10.0% by mass, yet still more preferably 5.0 to 10.0% by mass, and even yet still more preferably 6.0 to 9.5% by mass.

In the hair cosmetic of the present invention, from the viewpoint that a desired color shape can be imparted

during using as a temporary hair dye and that a lightness change on the hair with a bright color, such as bleached hair, is made small, the mass ratio [(A)/{(A) + (B)}] of the component (A) to the total content of the component (A) and the compound (B) is 0.30 or more, preferably 0.40 or more, more preferably 0.50 or more, and still more preferably 0.60 or more, and preferably 0.96 or less, preferably 0.94 or less, more preferably 0.92 or less, and still more preferably 0.90 or less. A specific range of the foregoing mass ratio [(A)/{(A) + (B)}] is 0.30 to 0.96, preferably 0.40 to 0.94, more preferably 0.50 to 0.92, and still more preferably 0.60 to 0.90.

<Component (C): Film-forming Polymer>

The hair cosmetic of the present invention contains a film-forming polymer as the component (C). In view of the fact that the hair cosmetic of the present invention contains the component (C), it is able to impart abrasion resistance, water resistance, removability by shampooing, softness, high color developability, and so on, and in the case where the hair cosmetic is a temporary hair dye, color transfer to the skin or clothing after hair dyeing is less, and a feeling on the hair can be made favorable.

Although the component (C) is not particularly restricted so long as it is a polymer capable of forming a film on the hair surface and is one which is typically used for the hair cosmetic, it is preferably a film-forming polymer having a silicone structure from the viewpoint that it is able to impart abrasion resistance, water resistance, removability by shampooing, softness, high color developability, and so on and that in the case where the hair cosmetic is a temporary hair dye, color transfer after hair dyeing is less, and a feeling on the hair is made favorable. The film-forming polymer having a silicone structure (hereinafter also referred to as "silicone structure-containing film-forming polymer") refers to a polymer not only having a silicone structure in at least a part thereof but also having a film-forming ability, and the "silicone structure" refers to a structure represented by the following general formula (I).

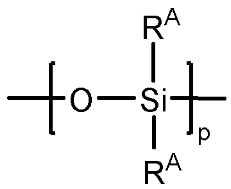

(I)

In the general formula (I), $R^A$'s are each independently a hydrocarbon group having 1 or more and 12 or less carbon atoms; and p is an integer of 1 or more. $R^A$ is preferably an alkyl group having 1 or more and 12 or less carbon atoms or an aryl group having 6 or more and 12 or less carbon atoms, more preferably an alkyl group having 1 or more and 12 or less carbon atoms or a phenyl group, still more preferably an alkyl group having 1 or more and 3 or less carbon atoms, and yet still more preferably a methyl group.

Although the silicone structure may be present in any of a main chain and a side chain in the polymer, it is preferably present in a main chain. In the case where the silicone structure is present in a polymer main chain, a binding mode thereof is not particularly restricted, and for example, the silicone structure may be present in an end of the polymer main chain, or may be a copolymerization polymer in which the silicone structure is bound in a block state or a random state in the polymer main chain. In addition, a polymer which has been graft modified with a compound having a silicone structure can also be used.

The silicone structure-containing film-forming polymer is preferably a poly(N-acylalkyleneimine)-modified organopolysiloxane from the viewpoint that it imparts abrasion resistance, water resistance, removability by shampooing, softness, high color develop ability, and so on and that in the case where the hair cosmetic is a temporary hair dye, color transfer after hair dyeing is less, and a feeling on the hair is made favorable. Specifically, the poly(N-acylalkyleneimine)-modified organopolysiloxane is an organopolysiloxane in which a poly(N-acylalkyleneimine) segment composed of a repeating unit represented by the following general formula (1) is bound to at least two silicon atoms of an organopolysiloxane segment constituting the main chain via a hetero atom-containing alkylene group.

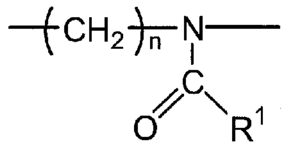

(1)

In the formula, $R^1$ represents a hydrogen atom, an alkyl group having 1 or more and 22 or less carbon atoms, an aralkyl group having 7 or more and 22 or less carbon atoms, or an aryl group having 6 or more and 22 or less carbon

atoms; and n represents 2 or 3.

Although at least two poly(N-acylalkyleneimine) segments can be bound to arbitrary silicon atoms constituting the organopolysiloxane segment via a hetero atom-containing alkylene group, it is preferably bound to at least one silicon atom excluding the both ends via the aforementioned alkyl group, and more preferably bound to at least two silicon atoms excluding the both ends via the aforementioned alkyl group.

Examples of the hetero atom-containing alkylene group mediating in the binding between the organopolysiloxane segment and the poly(N-acylalkyleneimine) include an alkylene group containing 1 to 3 of a nitrogen atom, an oxygen atom, and/or a sulfur atom and having 2 or more and 20 or less carbon atoms. Specific examples thereof include the following groups.

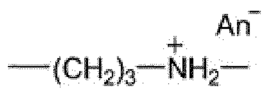

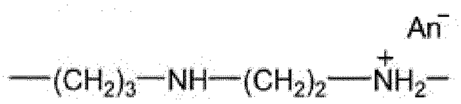

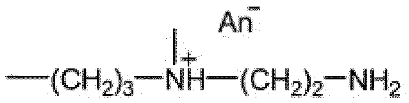

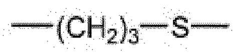

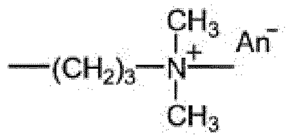

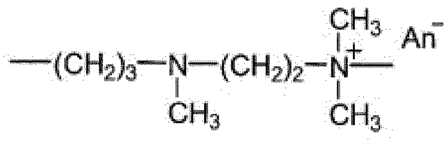

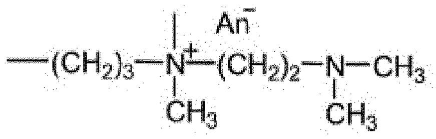

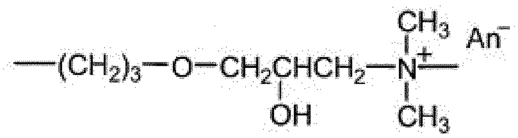

In the formulae, $An^-$ represents an anion.

An N-acylalkyleneimine unit constituting the poly(N-acylalkyleneimine) segment is one represented by the general formula (1).

Examples of the alkyl group having 1 or more and 22 or less carbon atoms in $R^1$ of the general formula (1) include linear, branched, or cyclic alkyl groups having 1 or more and 22 or less carbon atoms, and specifically, examples thereof include a methyl group, an ethyl group, an n-propyl group, an isopropyl group, an n-butyl group, a tert-butyl group, a pentyl group, a hexyl group, a cyclohexyl group, a heptyl group, an octyl group, a nonyl group, a decyl group, an undecyl group, a dodecyl group, an octadecyl group, a nonadecyl group, an eicosyl group, and a docosyl group. Of these, an alkyl group having 1 or more and 10 or less carbon atoms is preferred, an alkyl group having 1 or more and 6 or less carbon atoms is more preferred, and an alkyl group having 1 or more and 4 or less carbon atoms is still more preferred.

Examples of the aralkyl group having 7 or more and 22 or less carbon atoms in $R^1$ of the general formula (1) include a benzyl group, a phenethyl group, a trityl group, a naphthylmethyl group, and an anthracenylmethyl group. Of these, an aralkyl group having 7 or more and 14 or less carbon atoms is preferred, and an aralkyl group having 7 or more and 10 or less carbon atoms is more preferred.

Examples of the aryl group having 6 or more and 22 or less carbon atoms in $R^1$ of the general formula (1) include a phenyl group, a tolyl group, a xylyl group a naphthyl group, a biphenyl group, an anthryl group, and a phenanthryl group. Of these, an aryl group having 6 or more and 12 or less carbon atoms is preferred, and an aryl group having 6 or more and 9 or less carbon atoms is more preferred.

$R^1$ is preferably an alkyl group having 1 or more and 22 or less carbon atoms, more preferably an alkyl group having 1 or more and 10 or less carbon atoms, still more preferably an alkyl group having 1 or more and 6 or less carbon

atoms, and still more preferably an alkyl group having 1 or more and 4 or less carbon atoms.

Examples of the preferred poly(N-acylalkyleneimine)-modified organosiloxane include a poly(N-formylethyleneimine)organosiloxane, a poly(N-acetylethyleneimine)organosiloxane, and a poly(N-propionylethyleneimine)organosiloxane, and these can be used alone or in combination of two or more thereof.

Among those mentioned above, a poly(N-propionylethyleneimine)organosiloxane is preferred, and poly(N-propionylethyleneimine)methylsiloxane (N-propionylpolyethyleneimine/methylpolysiloxane copolymer; polysilicone-9) is more preferred from the viewpoint that it imparts abrasion resistance, water resistance, removability by shampooing, softness, high color developability, and so on and that in the case where the hair cosmetic is a temporary hair dye, color transfer after hair dyeing is less, and a feeling on the hair is made favorable.

As the component (C), a film-forming polymer not having a silicone structure other than those mentioned above can also be used, and examples of its classification include a cationic polymer, an anionic polymer, a nonionic polymer, and an amphoteric polymer.

Examples of the cationic polymer include a vinylpyrrohdone/dimethylaminoethyl methacrylate diethyl sulfate (e.g., H·C Polymer IS(M) and H·C Polymer 2 (both being available from Osaka Organic Chemical Industry Ltd.)), a vinylpyrrolidone/dimethylaminopropyl methacrylamide/lauryl dimethylaminopropyl methacrylamide copolymer (e.g., Styleze W-20 (available from Ashland)), polydimethylmethylenepiperidinium chloride (e.g., Marquat 100 (available from Lubrizol)), a dimethyldiallylammonium chloride/acrylamide copolymer (e.g., Marquat 550 (available from Lubrizol)), a vinylpyrrohdone/N,N-dimethylaminoethyl methacrylate copolymer diethyl sulfate (e.g., Gafquat 734 (available from Ashland)), a vinylpyrrohdone/dimethylaminoethyl methacrylate copolymer (e.g., Gafquat 440 (available from Ashland)), an N,N-dimethylaminoethyl methacrylate diethyl sulfate/N,N-dimethyl acrylamide/polyethylene glycol dimethacrylate copolymer (e.g., Sofcare KG-101W-E and Sofcare KG-301P (both being available from Kao Corporation)), ammonium-modified hydroxyethyl cellulose (e.g., a SoftCAT SL-30 polymer (available from The Dow Chemical Company)), and an acrylamide/DMAPA acrylate/methoxy methacrylate PEG copolymer.

Examples of the anionic polymer include an alkyl acrylate/diacetone acrylamide copolymer (e.g., Plascize L-9540B, Plascize L-53P, and Plascize L-9909B (all being available from Goo Chemical Co., Ltd.)), an alkyl acrylate/octylacrylamide copolymer (e.g., Dermacryl 79 (available from Akzo Nobel)), a vinyl acetate/crotonic acid/vinyl neodecanoate copolymer (e.g., RESYN 28-2930 (available from Akzo Nobel)), an acrylic acid/acrylamide/ethyl acrylate copolymer (e.g., Ultrahold 8 and Ultrahold Strong (both being available from BASF)), an alkyl acrylate copolymer (e.g., Aniset NF-1000 and Aniset HS-300 (both being available from Osaka Organic Chemistry Industry Ltd.)), a polyethylene glycol/polypropylene glycol-25/dimethicone/acrylate copolymer (e.g., Lubiflex SILK (available from BASF)), and an isophorone diisocyanate/dimethylolpropionic acid/(polyoxyethylene/polyoxypropylene) 4,4'-isopropylidenediphenol copolymer (e.g., DynamX (available from Akzo Nobel)).

Examples of the nonionic polymer include polyvinylpyrrolidone (e.g., Luviskol K17, Luviskol K30, and Luviskol K90 (all being available from BASF)), a vinylpyrrolidone/vinyl acetate copolymer (e.g., Luviskol VA73E and Luviskol 37E (both being available from BASF)), a vinyl methyl ether/alkyl maleate copolymer (e.g., Gantrez A-425 and Gantrez ES-225 (both being available from Ashland)), a vinylpyrrohdone/methacrylamide/vinylimidazole copolymer (e.g., Luviset Clear (available from BASF) and polyvinylcaprolactam (e.g., Luviskol Plus (available from BASF)), and Polysilicone-28.

Examples of the amphoteric polymer include an acrylates/lauryl acrylate/stearyl acrylate/ethylamine oxide methacrylate copolymer (e.g., Diaformer Z651 (available from Mitsubishi Chemical Corporation)), a methacryloyloxyethylcarboxybetaine/alkyl methacrylate copolymer (e.g., Yukaformer M75 and Yukaformer R205 (both being available from Mitsubishi Chemical Corporation)), an octylacrylamide/hydroxypropyl acrylate/butylaminoethyl methacrylate copolymer (e.g., Amphomer 28-4910 (available from Akzo Nobel)), and an octylacrylamide acrylate/hydroxypropyl acrylate/butylaminoethyl methacrylate copolymer (e.g., Amphomer SH30 (available from Akzo Nobel)).

The content of the component (C) in the hair cosmetic of the present invention is preferably 1% by mass or more, more preferably 2% by mass or more, still more preferably 3% by mass or more, and yet still more preferably 4% by mass or more from the viewpoint that in the case where the hair cosmetic is a temporary hair dye, color transfer after hair dyeing is less. In addition, the content of the component (C) in the hair cosmetic is preferably 30% by mass or less, more preferably 20% by mass or less, still more preferably 15% by mass or less, and yet still more preferably 12% by mass or less from the viewpoint that a feeling on the hair after hair dyeing is improved. A specific range of the content of the component (C) in the hair cosmetic is preferably 1 to 30% by mass, more preferably 2 to 20% by mass, still more preferably 3 to 15% by mass, and yet still more preferably 4 to 12% by mass.

In the hair cosmetic of the present invention, a mass ratio [(C)/(A)] of the component (C) to the component (A) is preferably 0.1 or more, more preferably 0.5 or more, still more preferably 0.8 or more, and yet still more preferably 1.0 or more from the viewpoint that in the case where the hair cosmetic is a temporary hair dye, color transfer after hair dyeing is less, and it is preferably 10 or less, more preferably 5 or less, still more preferably 3 or less, and yet still more preferably 2 or less from the viewpoint that a lightness change on the hair with a bright color, such as bleached hair, is made small. A specific range of the mass ratio [(C)/(A)] is preferably 0.1 to 10, more preferably 0.5 to 5, still more preferably 0.8 to 3, and yet still more preferably 1.0 to 2.

In the hair cosmetic of the present invention, the mass ratio [(C)/{(A) + (B)}] of the component (C) to the total content of the component (A) and the component (B) is 0.5 or more, preferably 0.6 or more, more preferably 0.7 or more, and still more preferably 0.8 or more from the viewpoint that in the case where the hair cosmetic is a temporary hair dye, color transfer after hair dyeing is less, and it is 2.4 or less, preferably 2.2 or less, more preferably 2.0 or less, still more preferably 1.4 or less, and yet still more preferably 1.2 or less from the viewpoint that a feeling on the hair after hair dyeing is improved. A specific range of the mass ratio [(C)/{(A) + (B)}] is 0.5 to 2.4, preferably 0.5 to 2.2., more preferably 0.5 to 2.0, still more preferably 0.5 to 1.4, yet still more preferably 0.6 to 1.4, even yet still more preferably 0.7 to 1.4, even still more preferably 0.7 to 1.2, and even still more further preferably 0.8 to 1.2.

<Component (D): Nonaqueous Solvent>

The hair cosmetic of the present invention contains, as the component (D), a nonaqueous solvent for the purpose of dispersing or dissolving the components (A) to (C) and other component. The nonaqueous solvent is a solvent other than water, and from the viewpoint of dispersing or dissolving the respective components in the hair cosmetic and the viewpoint of improving applicability and a drying rate after application, a volatile polar solvent is preferably contained.
From the aforementioned viewpoint, examples of the volatile polar solvent include at least one selected from the group consisting of an alcohol, an ester, and a ketone, each having preferably 6 or less carbon atoms, and more preferably 4 or less carbon atoms, and it is preferred to contain an alcohol having 4 or less carbon atoms. Examples of the alcohol having 4 or less carbon atoms include ethanol, 1-propanol, 2-propanol, 1-butanol, and 2-butanol. Among the foregoing alcohols, at least one selected from the group consisting of ethanol and 2-propanol is more preferred, and from the viewpoint of improving a drying rate after application, ethanol is still more preferred.
The content of the at least one selected from the group consisting of ethanol and 2-propanol in the component (D) is preferably 30% by mass or more, more preferably 50% by mass or more, still more preferably 80% by mass or more, and yet still more preferably 85% by mass or more, and an upper limit thereof is 100% by mass from the viewpoint that applicability and a drying rate after application are improved and the viewpoint that in the case where the hair cosmetic is a temporary hair dye, a lightness change on the hair with a dark color, such as black hair, and on the hair with a bright color, such as bleached hair, is made small. In addition, the content of ethanol in the component (D) is preferably 30% by mass or more, more preferably 50% by mass or more, and still more preferably 80% by mass or more, and an upper limit thereof is 100% by mass from the viewpoint that applicability and a drying rate after application are improved and the viewpoint that in the case where the hair cosmetic is a temporary hair dye, a lightness change on the hair with a dark color, such as black hair, and on the hair with a bright color, such as bleached hair, is made small.
The content of the component (D) in the hair cosmetic of the present invention is preferably 30% by mass or more, more preferably 50% by mass or more, and still more preferably 70% by mass or more, and preferably 95% by mass or less from the viewpoint of dispersing or dissolving the respective components in the hair cosmetic and the viewpoint of improving applicability and a drying rate after application.
In the hair cosmetic of the present invention, from the viewpoint that drying after application is fast, and handling properties during use are favorable and the viewpoint that in the case where the hair cosmetic is a temporary hair dye, a lightness change on the hair with a bright color, such as bleached hair, is made small, the content of water is 40% by mass or less, preferably 30% by mass or less, more preferably 20% by mass or less, still more preferably 15% by mass or less, yet still more preferably 10% by mass or less, even yet still more preferably 5% by mass or less, even still more preferably 2% by mass or less, and even still more further preferably 1% by mass or less, and an lower limit thereof is 0% by mass.

<Silicone Oil>

From the viewpoint of improving dispersibility of the component (C) and the viewpoint that in the case where the hair cosmetic is a temporary hair dye, a feeling on the hair after hair dyeing is improved, the hair cosmetic of the present invention can further contain a silicone oil.
As for the silicone oil, there are preferably exemplified a phenyl-modified silicone and a dimethyl silicone, each of which has a dynamic viscosity at 25°C of 200 $mm^2/s$ or less.
Examples of the phenyl-modified silicone include methylphenyl polysiloxane (diphenyl dimethicone), phenyl methicone, and phenyl trimethicone. Specifically, examples thereof include KF-50-100cs (dynamic viscosity at 25°C: 100 $mm^2/s$), KF-53 (dynamic viscosity at 25°C: 175 $mm^2/s$), and KF-56 (dynamic viscosity at 25°C: 14 $mm^2/s$) (all being available from Shin-Etsu Chemical Co., Ltd.); and SH 556 FLUID (dynamic viscosity at 25°C: 14 $mm^2/s$) (available from Dow Corning Toray Co., Ltd.).
Examples of the dimethyl silicone include dimethyl polysiloxane (dimethicone). Specifically, examples thereof include KF-96A-200cs (dynamic viscosity at 25°C: 200 $mm^2/s$), KF-96A-100cs (dynamic viscosity at 25°C: 100 $mm^2/s$), KF-96A-50cs (dynamic viscosity at 25°C: 50 $mm^2/s$), KF-96A-30cs (dynamic viscosity at 25°C: 30 $mm^2/s$), KF-96A-20cs

(dynamic viscosity at 25°C: 20 $mm^2/s$), KF-96A-10cs (dynamic viscosity at 25°C: 10 $mm^2/s$), KF-96A-6cs (dynamic viscosity at 25°C: 6 $mm^2/s$), KF-96A-5cs (dynamic viscosity at 25°C: 5 $mm^2/s$), KF-96L-2cs (dynamic viscosity at 25°C: 2 $mm^2/s$), KF-96L-1.5cs (dynamic viscosity at 25°C: 1.5 $mm^2/s$), and KF-96L-1cs (dynamic viscosity at 25°C: 1 $mm^2/s$) (all being available from Shin-Etsu Chemical Co., Ltd.); and SH200 C Fluid 200cs (dynamic viscosity at 25°C: 200 $mm^2/s$), SH200 C Fluid 100cs (dynamic viscosity at 25°C: 100 $mm^2/s$), SH200 C Fluid 50cs (dynamic viscosity at 25°C: 50 $mm^2/s$), SH200 C Fluid 30cs (dynamic viscosity at 25°C: 30 $mm^2/s$), SH200 C Fluid 20cs (dynamic viscosity at 25°C: 20 $mm^2/s$), SH200 C Fluid lOcs (dynamic viscosity at 25°C: 10 $mm^2/s$), SH200 C Fluid 6cs (dynamic viscosity at 25°C: 6 $mm^2/s$), SH200 C Fluid 5cs (dynamic viscosity at 25°C: 5 $mm^2/s$), SH200 C Fluid 2cs (dynamic viscosity at 25°C: 2 $mm^2/s$), SH200 C Fluid 1.5cs (dynamic viscosity at 25°C: 1.5 $mm^2/s$), and SH200 C Fluid 1cs (dynamic viscosity at 25°C: 1 $mm^2/s$) (all being available from Dow Corning Toray Co., Ltd.).

From the viewpoint of improving dispersibility of the component (C) and the viewpoint that in the case where the hair cosmetic is a temporary hair dye, a feeling on the hair after hair dyeing is improved, the dynamic viscosity at 25°C of the silicone oil is preferably 200 $mm^2/s$ or less, more preferably 150 $mm^2/s$ or less, and still more preferably 100 $mm^2/s$ or less. The dynamic viscosity can be measured at 25°C with an Ubbelohde viscometer on the basis of JIS Z8803: 2011 "Methods for viscosity measurement of liquid".

The silicone oil can be used alone or in combination of two or more thereof.

From the viewpoint of improving dispersibility of the component (C) and the viewpoint that in the case where the hair cosmetic is a temporary hair dye, a feeling on the hair after hair dyeing is improved, the content of the silicone oil in the hair cosmetic is preferably 0.1% by mass or more, more preferably 0.5% by mass or more, and still more preferably 1% by mass or more, and preferably 20% by mass or less, more preferably 15% by mass or less, and still more preferably 10% by mass or less. A specific range of the content of the silicone oil in the hair cosmetic is preferably 0.1 to 20% by mass, more preferably 0.5 to 15% by mass, and still more preferably 1 to 10% by mass.

<Other Component>

To the hair cosmetic of the present invention, a component which is typically used for the hair cosmetic can be added in addition to the aforementioned components. Examples of the foregoing component include a surfactant; an oil, such as oils and fats and a sparingly volatile hydrocarbon; a thickener; a propellant, such as LPG (liquefied petroleum gas), pentane, dimethyl ether, and diethyl ether; and others, such as a perfume, an antiseptic, an ultraviolet absorber, a chelating agent, an antioxidant, and a vegetable extract.

A production method of the hair cosmetic of the present invention is not particularly restricted, and the hair cosmetic can be produced by stirring and mixing the aforementioned respective components by using a known device.

<Formulation and Product Form>

The formulation of the hair cosmetic of the present invention is not particularly restricted, and for example, in the case where the hair cosmetic is a temporary hair dye, examples of the formulation thereof include a foam, a gel, a spray, a cream, and a wax. From the viewpoint of applying a temporary hair dye to a target site of the hair by using an application tool, such as a brush, a comb, and a sponge, it is preferred that the temporary hair dye is in a liquid form in a viscosity range as mentioned later.

The product form of the temporary hair dye is preferably of a mascara type. When the product form is of a mascara type, the temporary hair dye can be easily applied in the target site of the hair by using an application tool, such as a brush and a sponge. In addition, since the component (A) and the component (B) which are high in the specific gravity are liable to precipitate in the liquid of the temporary hair dye, the mascara-type temporary hair dye can be used through redispersion by shaking a mascara container by hand prior to application, and it is excellent in handling properties and suitable from the standpoint of portability.

In the case where the hair cosmetic is a temporary hair dye, its viscosity at 25°C may be more than 0 mPa·s from the viewpoint of minimizing liquid dripping during the application operation, and the foregoing viscosity at 25°C is preferably 1 mPa·s or more, more preferably 5 mPa·s or more, and still more preferably 10 mPa·s or more from the viewpoint of suppressing liquid dripping and splash of liquid during the application operation, and it is preferably 3,000 mPa·s or less, more preferably 2,000 mPa·s or less, still more preferably 1,500 mPa·s or less, yet still more preferably 1,000 mPa·s or less, even yet still more preferably 800 mPa·s or less, even still more preferably 500 mPa·s or less, even still further preferably 300 mPa·s or less, and even yet still more further preferably 150 mPa·s or less from the viewpoint of increasing the holding amount on an applied body and evenly applying in a single operation from the root to the tip of the hair without causing liquid outage.

The aforementioned viscosity is a value when measured at a temperature of 25±1°C for 1 minute at a predetermined rotation rate with a B-type viscometer using a rotor according to the measuring adaptive range. Specifically, 70 to 80 g of the hair cosmetic is charged in a screw tube having a capacity of 100 to 200 mL and shaken by hand 100

times in a width of about 30 cm for 30 seconds such that it becomes uniform; further, immediately after standing up 30 times at a speed of one time per second, using a B-type viscometer (TV-10M, available from Toki Sangyo Co., Ltd.), the viscosity is measured under conditions of using a rotor No. M1 for up to viscosities of 100 mPa·s at a rotation speed of 100 rpm, using a rotor No. M2 in a range of 100 to 500 mPa·s at a rotation speed of 60 rpm, using a rotor No. M3 in a range of 500 to 2,000 mPa·s at a rotation speed of 60 rpm, and using a rotor No. M4 in a range of 2,000 to 10,000 mPa·s at a rotation speed of 60 rpm.

[Use]

The present invention also provides use of, a hair cosmetic, a composition containing the following components (A) to (D):

(A) an interference pearl pigment,
(B) other pigment than the component (A),
(C) a film-forming polymer, and
(D) a nonaqueous solvent,

wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water being 40% by mass or less. The foregoing hair cosmetic is preferably a temporary hair dye.

The components contained in the aforementioned composition and preferred embodiments thereof are the same as those in the aforementioned hair cosmetic.

For example, in the case where the aforementioned composition and hair cosmetic are a temporary hair dye, as for the use method thereof, after applying the temporary hair dye on the hair through application, etc., it is allowed to stand and used without rinsing away.

With respect to the aforementioned embodiments, the present invention discloses the following hair cosmetic, use of composition, and hair dyeing method.

<1> A hair cosmetic containing the following components (A) to (D):

(A) an interference pearl pigment,
(B) other pigment than the component (A),
(C) a film-forming polymer, and
(D) a nonaqueous solvent,

wherein,

the total content of the component (A) and the component (B) is 2.0% by mass or more and 12.0% by mass or less,
a mass ratio [(A)/{(A) + (B)}] of the component (A) to the total content of the component (A) and the component (B) is 0.30 or more and 0.96 or less,
a mass ratio [(C)/{(A) + (B)}] of the component (C) to the total content of the component (A) and the component (B) is 0.5 or more and 2.4 or less, and
the content of water is 40% by mass or less, preferably 30% by mass or less, more preferably 20% by mass or less, still more preferably 15% by mass or less, yet still more preferably 10% by mass or less, even yet still more preferably 5% by mass or less, even still more preferably 2% by mass or less, and even still more further preferably 1% by mass or less.

<2> The hair cosmetic as set forth in <1>, wherein the component (D) contains ethanol in an amount of 30% by mass or more, preferably 50% by mass or more, and more preferably 70% by mass or more, and 100% by mass or less.
<3> The hair cosmetic as set forth in <1> or <2>, wherein the total content of the component (A) and the component (B) is preferably 3.0% by mass or more, more preferably 3.5% by mass or more, still more preferably 4.0% by mass or more, yet still more preferably 5.0% by mass or more, and even yet still more preferably 6.0% by mass or more, and preferably 11.0% by mass or less, more preferably 10.0% by mass or less, and still more preferably 9.5% by mass or less.
<4> The hair cosmetic as set forth in any one of <1> to <3>, wherein the mass ratio [(A)/{(A) + (B)}] of the component (A) to the total content of the component (A) and the component (B) is preferably 3.0% by mass or more, more

preferably 3.5% by mass or more, still more preferably 4.0% by mass or more, yet still more preferably 5.0% by mass or more, and even yet still more preferably 6.0% by mass or more, and preferably 11.0% by mass or less, more preferably 10.0% by mass or less, and still more preferably 9.5% by mass or less.

<5> The hair cosmetic as set forth in any one of <1> to <4>, wherein the mass ratio [(C)/{(A) + (B)}] of the component (C) to the total content of the component (A) and the component (B) is preferably 0.6 or more, more preferably 0.7 or more, and still more preferably 0.8 or more, and preferably 2.2 or less, more preferably 2.0 or less, still more preferably 1.4 or less, and yet still more preferably 1.2 or less.

<6> A hair cosmetic containing the following components (A) to (D):

(A) an interference pearl pigment,
(B) other pigment than the component (A),
(C) a film-forming polymer, and
(D) a nonaqueous solvent,

wherein,

the total content of the component (A) and the component (B) is 2.0% by mass or more and 12.0% by mass or less,
a mass ratio [(A)/{(A) + (B)}] of the component (A) to the total content of the component (A) and the component (B) is 0.30 or more and 0.96 or less,
a mass ratio [(C)/{(A) + (B)}] of the component (C) to the total content of the component (A) and the component (B) is 0.5 or more and 1.4 or less,
the component (D) contains ethanol in an amount of 30% by mass or more, and
the content of water is 10% by mass or less.

<7> A hair cosmetic containing the following components (A) to (D):

(A) an interference pearl pigment,
(B) other pigment than the component (A),
(C) a film-forming polymer, and
(D) a nonaqueous solvent,

wherein,

the total content of the component (A) and the component (B) is 4.0% by mass or more and 10.0% by mass or less,
a mass ratio [(A)/{(A) + (B)}] of the component (A) to the total content of the component (A) and the component (B) is 0.30 or more and 0.96 or less,
a mass ratio [(C)/{(A) + (B)}] of the component (C) to the total content of the component (A) and the component (B) is 0.5 or more and 1.4 or less,
the component (D) contains ethanol in an amount of 30% by mass or more, and
the content of water is 10% by mass or less.

<8> The hair cosmetic as set forth in any one of <1> to <7>, wherein an absolute value of a hue difference ΔH° measured by the following method (I) is preferably 30 or less, more preferably 15 or less, and still more preferably 10 or less.

Method (I): With respect to the hair cosmetic, a sample A containing only the component (A) as the pigment (a sample in which the component (B) is not blended in the hair cosmetic, and the component (B) is replaced by the same amount of ethanol) and a sample B containing only the component (B) as the pigment (a sample in which the component (A) is not blended in the hair cosmetic, and the component (A) is replaced by the same amount of ethanol) are prepared. 2 g of the sample A is uniformly applied on a black plate of 2 cm × 2 cm and 2 g of the sample B is uniformly applied on a white plate of 2 cm × 2 cm, respectively and allowed to stand for natural drying at 25°C for 1 hour. A hue of the sample-applied surface of each of the plates is measured with a color-difference meter, and the absolute value of the hue difference ΔH° is determined from a difference therebetween.

<9> The hair cosmetic as set forth in any one of <1> to <8>, wherein the component (C) is a film-forming polymer having a silicone structure; preferably a poly(N-acylalkyleneimine)-modified organopolysiloxane; more preferably an organopolysiloxane in which a poly(N-acylalkyleneimine) segment composed of a repeating unit represented by the following general formula (1) is bound to at least two silicon atoms of an organopolysiloxane segment constituting the main chain via a hetero atom-containing alkylene group; still more preferably one or more selected from the group consisting of poly(N-formylethyleneimine)organosiloxane, a poly(N-acetylethyleneimine)organosiloxane, and

a poly(N-propionylethyleneimine)organosiloxane; yet still more preferably a poly(N-propionylethyleneimine)organosiloxane; and even yet still more preferably poly(N-propionylethyleneimine)methylsiloxane:

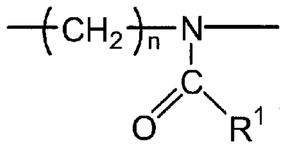

(1)

wherein $R^1$ represents a hydrogen atom, an alkyl group having 1 or more and 22 or less carbon atoms, an aralkyl group having 7 or more and 22 or less carbon atoms, or an aryl group having 6 or more and 22 or less carbon atoms; and n represents 2 or 3.

<10> The hair cosmetic as set forth in any one of <1> to <9>, wherein a mass ratio [(C)/(A)] of the component (C) to the component (A) is preferably 0.1 or more, more preferably 0.5 or more, still more preferably 0.8 or more, and yet still more preferably 1.0 or more, and preferably 10 or less, more preferably 5 or less, still more preferably 3 or less, and yet still more preferably 2 or less.

<11> The hair cosmetic as set forth in any one of <6> to <10>, wherein the mass ratio [(C)/{(A) + (B)}] of the component (C) to the total content of the component (A) and the component (B) is preferably 0.6 or more, more preferably 0.7 or more, and still more preferably 0.8 or more, and preferably 1.2 or less.

<12> The hair cosmetic as set forth in any one of <1> to <11>, wherein the component (A) is a pigment exhibiting a pearly luster owing to a reflection and interference action of light and having a coating layer on a surface of a core particle, the coating layer including a reflection and interference layer and having translucency, and the content of a colored metal oxide and a colored metal in the coating layer is preferably 3% by mass or less, more preferably 1% by mass or less, and still more preferably 0.1% by mass or less.

<13> The hair cosmetic as set forth in <12>, wherein the colored metal oxide is at least one selected from the group consisting of iron oxide, copper oxide, cobalt oxide, chromium oxide, and nickel oxide, and the colored metal is at least one selected from the group consisting of gold and copper.

<14> The hair cosmetic as set forth in any one of <1> to <13>, wherein a material of the core particle constituting the component (A) is at least one selected from the group consisting of mica, sericite, talc, kaolin, a smectite group clay mineral, bismuth oxychloride, synthetic mica, synthetic sericite, tabular silica, and tabular alumina, and preferably mica.

<15> The hair cosmetic as set forth any one of <12> to <14>, wherein the reflection and interference layer included in the coating layer of the component (A) is a layer formed of a thin film composed mainly of a colorless metal oxide or a colorless metal; preferably a layer formed of a thin film composed mainly of at least one selected from the group consisting of titanium dioxide, zinc oxide, aluminum oxide, titanium, zirconium, zinc, tin, silicon, and aluminum; more preferably a layer formed of a thin film composed mainly of at least one selected from the group consisting of titanium and titanium dioxide; and still more preferably a layer formed of a thin layer composed of mainly of titanium dioxide.

<16> The hair cosmetic as set forth in any one of <1> to <15>, wherein the component (A) is titanium dioxide-coated mica (micaceous titanium).

<17> The hair cosmetic as set forth in any one of <12> to <16>, wherein a ratio between the core particle and the reflection and interference layer constituting the component (A) is preferably 25/75 to 80/20, more preferably 30/70 to 70/30, and still more preferably 50/50 to 65/35 in terms of a mass ratio of the core particle to the colorless metal oxide and the colorless metal constituting the reflection and interference layer [(core particle)/(colorless metal oxide and colorless metal constituting the reflection and interference layer)].

<18> The hair cosmetic as set forth in any one of <1> to <17>, wherein the content of the component (A) is preferably 1.5 to 10% by mass, more preferably 2.5 to 10% by mass, still more preferably 3.0 to 9.5% by mass, yet still more preferably 3.5 to 9.0% by mass, and even yet still more preferably 4.0 to 8.5% by mass.

<19> The hair cosmetic as set forth in any one of <1> to <18>, wherein the content of the component (B) is preferably 0.5% by mass or more, more preferably 0.8% by mass or more, and still more preferably 1.0% by mass or more, and preferably 8.0% by mass or less, more preferably 7.0% by mass or less, still more preferably 6.0% by mass or less, yet still more preferably 5.0% by mass or less, and even yet still more preferably 4.0% by mass or less.

<20> The hair cosmetic as set forth in any one of <1> to <19>, wherein the content of the component (C) is preferably 1% by mass or more, more preferably 2% by mass or more, still more preferably 3% by mass or more, and yet still more preferably 4% by mass or more, and preferably 30% by mass or less, more preferably 20% by mass or less, still more preferably 15% by mass or less, and yet still more preferably 12% by mass or less.

<21> The hair cosmetic as set forth in any one of <1> to <20>, wherein the content of the component (D) is preferably 30% by mass or more, more preferably 50% by mass or more, and still more preferably 70% by mass or more, and preferably 95% by mass or less.

<22> The hair cosmetic as set forth in any one of <1> to <21>, further containing a silicone oil in an amount of preferably 0.1% by mass or more, more preferably 0.5% by mass or more, and still more preferably 1% by mass or more, and preferably 20% by mass or less, more preferably 15% by mass or less, and still more preferably 10% by mass or less.
<23> The hair cosmetic as set forth in any one of <1> to <22>, which is a temporary hair dye, and preferably a temporary hair dye of a mascara type in terms of a product form.
<24> The hair cosmetic as set forth in <23>, wherein a viscosity at 25°C is more than 0 mPa·s, preferably 1 mPa·s or more, more preferably 5 mPa·s or more, and still more preferably 10 mPa·s or more, and preferably 3,000 mPa·s or less, more preferably 2,000 mPa·s or less, still more preferably 1,500 mPa·s or less, yet still more preferably 1,000 mPa·s or less, even yet still more preferably 800 mPa·s or less, even still more preferably 500 mPa·s or less, even still further preferably 300 mPa·s or less, and even yet still more further preferably 150 mPa·s or less.
<25> Use of a composition as a hair cosmetic, the composition containing the following components (A) to (D):

(A) an interference pearl pigment,
(B) other pigment than the component (A),
(C) a film-forming polymer, and
(D) a nonaqueous solvent,

wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water is 40% by mass or less, preferably 30% by mass or less, more preferably 20% by mass or less, still more preferably 15% by mass or less, yet still more preferably 10% by mass or less, even yet still more preferably 5% by mass or less, even still more preferably 2% by mass or less, and even still more further preferably 1% by mass or less.
<26> A hair dyeing method including applying hair with a composition containing the following components (A) to (D):

(A) an interference pearl pigment,
(B) other pigment than the component (A),
(C) a film-forming polymer, and
(D) a nonaqueous solvent,

wherein the total content of the component (A) and the component (B) is 2.0% by mass or more and 12.0% by mass or less; a mass ratio [(A)/{(A) + (B)}] of the component (A) to the total content of the component (A) and the component (B) is 0.30 or more and 0.96 or less; a mass ratio [(C)/{(A) + (B)}] of the component (C) to the total content of the component (A) and the component (B) is 0.5 or more and 2.4 or less; and the content of water is 40% by mass or less, preferably 30% by mass or less, more preferably 20% by mass or less, still more preferably 15% by mass or less, yet still more preferably 10% by mass or less, even yet still more preferably 5% by mass or less, even still more preferably 2% by mass or less, and even still more further preferably 1% by mass or less.

Examples

The present invention is hereunder described by reference to Examples, but it should be construed that the present invention is not limited to the scope of the Examples.

Examples 1 to 10 and Comparative Examples 1 to 7 (Preparation and Evaluation of Temporary Hair Dye)

A mixture of the component (A) and the component (B), or the component (A), as shown in Table 1 was added to the component (D) or water, followed by stirring with a disper disperser at 1,000 rpm for 5 minutes. To the resultant, the component (C) and the remaining component(s) were added and then stirred with a disper disperser at 1,000 rpm for 5 minutes, to obtain a temporary hair dye of each of the Examples. Using the obtained temporary hair dye, various evaluations were performed by the methods as mentioned later.
In the temporary hair dyes of Examples 1 and 2, a sample A containing only the component (A) as the pigment (a sample in which the component (B) was not blended in the temporary hair dye, and the component (B) was replaced by the same amount of ethanol) and a sample B containing only the component (B) as the pigment (a sample in which the component (A) was not blended in the temporary hair dye, and the component (A) was replaced by the same amount of ethanol) were prepared, and an absolute value of a hue difference $\Delta H°$ was measured by the following method.
2 g of the sample A was uniformly applied on a black plate of 2 cm $\times$ 2 cm and 2 g of the sample B was uniformly

applied on a white plate of 2 cm × 2 cm, respectively and allowed to stand for natural drying at 25°C for 1 hour. A hue of the sample-applied surface of each of the plates was measured with a color-difference meter (CR-400, available from Konica Minolta, Inc.), and the hue difference ΔH° was determined from a difference therebetween.

In the temporary hair dye of Example 1, a hue h1 of the sample A was 280.25, and a hue h2 of the sample B was 270.52, and thus, the absolute value of the hue difference ΔH° was 9.73. In addition, in the temporary hair dye of Example 2, a hue h1 of the sample A was 25.02, and a hue h2 of the sample B was 20.90, and thus, the absolute value of the hue difference ΔH° was 4.12.

<Hair Dyeing Properties>

Two kinds of tresses which are a black hair tress having a length of 10 cm and a mass of 1 g (BS-B-A, available from Beaulax Co., Ltd.) and a bleached tress prepared by bleaching the foregoing black hair tress into 9 tones (in a range where the lightness L* is 26.9 to 30.4, and the chroma C* is 18.0 to 20.6) were prepared. The respective tresses were washed once with a plain hair shampoo having the following composition, air-dried, and then uniformly applied with 0.2 g of the temporary hair dye obtained in each of the Examples to perform hair dyeing, followed by allowing to stand for natural drying at 25°C for 30 minutes.

With respect to each of the black hair tress and the bleached tress, the tress before hair dyeing and after the aforementioned hair dyeing was measured in the CIE standard colorimetric system (L*,a*,b*) by using a color-difference meter (CR-400, available from Konica Minolta, Inc.), and a color difference ΔE* and a lightness difference ΔL* were calculated according to the following equations. The measurement of L*,a*,b* was performed at three different points on the tress (every one point in the center of each of three areas resulting through trisection of the tress in the lengthwise direction), and an average value thereof was calculated.

So long as in the black hair tress, ΔE* is 5 or more, and ΔL* is within ±5, and preferably ΔE* is 6 or more, and ΔL* is within ±5, and in the bleached tress, ΔE* is 10 or more, and ΔL* is within ±10, and preferably ΔE* is 10 or more, and ΔL* is within ±5, not only the color change after hair dyeing can be actually felt, but also the color shape is not excessively graudy, and the hair dyeing properties are favorable.

$$\Delta E^* = \sqrt{(L_1^* - L_0^*)^2 + (a_1^* - a_0^*)^2 + (b_1^* - b_0^*)^2}$$

$L_0^*, a_0^*, b_0^*$: measured value of the tress before hair dyeing
$L_1^*, a_1^*, b_1^*$: measured value of the tress after hair dyeing

$$\Delta L^* = L_1^* - L_0^*$$

$L_0^*$: lightness of the tress before hair dyeing
$L_1^*$: lightness of the tress after hair dyeing

[Composition of Plain Shampoo]

| Component | (% by mass) |
|---|---|
| Sodium polyoxyethylene(2) lauryl ether sulfate (*1) | 15.5 |
| Lauric acid diethanolamide (*2) | 1.5 |
| Tetrasodium edetate | 0.3 |
| Sodium benzoate | 1.43 |
| Purified water | Balance |
| Total | 100.0 |

*1: 42.0% by mass as EMAL 227 (available from Kao Corporation, active ingredient: 27% by mass)
*2: AMINON L-02 (available from Kao Corporation)

<Drying Rate>

By applying a black hair tress having a length of 10 cm and a mass of 1 g (BS-B-A, available from Beaulax

Co., Ltd.) with 0.2 g of the temporary hair dye obtained in each of the Examples, allowing to stand at 25°C, and pressing against Kimwipes every one minute by using a 100-g weight, a time until soaking of the temporary hair dye into the Kimwipes was not confirmed was evaluated.

<Smallness of Color Transfer after Hair Dyeing>

A black hair tress having a length of 10 cm and a mass of 1 g (BS-B-A, available from Beaulax Co., Ltd.) was applied with 0.2 g of the temporary hair dye obtained in each of the Examples and allowed to stand for natural drying at 25°C for 30 minutes. The aforementioned tress was sandwiched by Kimwipes, the tress was pulled out while applying a load by using a 200-g weight, and a degree of color transfer to the Kimwipes was visually observed, thereby evaluating the smallness of color transfer according to the following evaluation criteria. An average value of evaluation scores by five expert panelists (rounded off from the first decimal place) is shown in Table 1.

(Evaluation Criteria and Score)

1: No color transfer is observed.
2: Color transfer is slightly observed, but it is within the tolerance.
3: Color transfer is significantly observed.

<Feeling on Hair after Hair Dyeing>

The hair of each of five expert panelists was applied with 0.2 g of the temporary hair dye obtained in each of the Examples and allowed to stand for natural drying at 25°C for 30 minutes. Thereafter, the feeling (non-stiffness) on the hair was evaluated according to the following evaluation criteria. An average value of evaluation scores by five expert panelists (rounded off from the first decimal place) is shown in Table 1.

(Evaluation Criteria and Score)

1: The feeling is favorable.
2: The feeling is slightly good.
3: The feeling is no good.

Table 1

| (% by mass) | | Example | | | | | | | | | | Comparative Example | | | | | | |
|---|---|---|---|---|---|---|---|---|---|---|---|---|---|---|---|---|---|---|
| | | 1 | 2 | 3 | 4 | 5 | 6 | 7 | 8 | 9 | 10 | 1 | 2 | 3 | 4 | 5 | 6 | 7 |
| (A) | Micaceous titanium A *1 | 6.5 | | 8.0 | 4.6 | 2.6 | 6.5 | 6.5 | 6.5 | 6.5 | 6.5 | 9.0 | 1.5 | 1.3 | 9.1 | 6.5 | 6.5 | 6.5 |
| | Micaceous titanium B *2 | | 6.5 | | | | | | | | | | | | | | | |
| (B) | Yellow No. 401/barium sulfate *3 | 0.63 | 0.41 | 0.25 | 1.09 | 0.25 | 0.63 | 0.63 | 0.63 | 0.63 | 0.63 | | 1.87 | 0.13 | 0.89 | 0.63 | 0.63 | 0.63 |
| | Red No. 226 *4 | 0.95 | 2.03 | 0.38 | 1.64 | 0.38 | 0.95 | 0.95 | 0.95 | 0.95 | 0.95 | | 2.81 | 0.19 | 1.33 | 0.95 | 0.95 | 0.95 |
| | Blue No. 404 *5 | 0.95 | 0.10 | 0.38 | 1.64 | 0.38 | 0.95 | 0.95 | 0.95 | 0.95 | 0.95 | | 2.81 | 0.19 | 1.33 | 0.95 | 0.95 | 0.95 |
| (C) | Polysilicone-9 *6 | 10.2 | 10.2 | 10.2 | 10.2 | 3.1 | 10.2 | 10.2 | 13.4 | 18.1 | 5.4 | 10.2 | 10.2 | 1.6 | 11.0 | 10.2 | 2.7 | 22.5 |
| (D) | Ethanol | 75.76 | 75.76 | 75.78 | 75.83 | 91.28 | 19.80 | 60.76 | 72.56 | 67.86 | 80.54 | 75.80 | 75.81 | 95.59 | 69.35 | 19.80 | 83.25 | 63.46 |
| | Isopropanol | | | | | | 55.96 | | | | | | | | | | | |
| Methylphenyl polysiloxane *7 | | 5.0 | 5.0 | 5.0 | 5.0 | 2.0 | 5.0 | 5.0 | 5.0 | 5.0 | 5.0 | 5.0 | 5.0 | 1.0 | 7.0 | 5.0 | 5.0 | 5.0 |
| Water | | | | | | | | 15.00 | | | | | | | | 55.96 | | |
| Total | | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| Content of component (A) (% by mass) | | 6.5 | 6.5 | 8.0 | 4.6 | 2.6 | 6.5 | 6.5 | 6.5 | 6.5 | 6.5 | 9.0 | 1.5 | 1.3 | 9.1 | 6.5 | 6.5 | 6.5 |
| Content of component (B) (% by mass) | | 2.54 | 2.54 | 1.02 | 4.37 | 1.02 | 2.54 | 2.54 | 2.54 | 2.54 | 2.54 | 0.00 | 7.49 | 0.51 | 3.55 | 2.54 | 2.54 | 2.54 |
| Total content of components (A) and (B) (% by mass) | | 9.04 | 9.04 | 9.02 | 8.97 | 3.62 | 9.04 | 9.04 | 9.04 | 9.04 | 9.04 | 9.00 | 8.99 | 1.81 | 12.65 | 9.04 | 9.04 | 9.04 |
| Content of component (C) (% by mass) | | 10.2 | 10.2 | 10.2 | 10.2 | 3.1 | 10.2 | 10.2 | 13.4 | 18.1 | 5.4 | 10.2 | 10.2 | 1.6 | 11.0 | 10.2 | 2.7 | 22.5 |
| Content of component (D) (% by mass) | | 75.76 | 75.76 | 75.78 | 75.83 | 91.28 | 75.76 | 60.76 | 72.56 | 67.86 | 80.54 | 75.80 | 75.81 | 95.59 | 69.35 | 19.80 | 83.25 | 63.46 |
| Mass ratio [(A)/{(A) + (B)}] | | 0.72 | 0.72 | 0.89 | 0.51 | 0.72 | 0.72 | 0.72 | 0.72 | 0.7 | 0.7 | 1.00 | 0.17 | 0.72 | 0.72 | 0.72 | 0.7 | 0.7 |
| Mass ratio [(C)/(A)] | | 1.6 | 1.6 | 1.3 | 2.2 | 1.2 | 1.6 | 1.6 | 2.1 | 2.8 | 0.8 | 1.1 | 6.8 | 1.2 | 1.2 | 1.6 | 0.4 | 3.5 |
| Mass ratio [(C)/{(A) + (B)}] | | 1.1 | 1.1 | 1.1 | 1.1 | 0.9 | 1.1 | 1.1 | 1.5 | 2.0 | 0.6 | 1.1 | 1.1 | 0.9 | 0.9 | 1.1 | 0.3 | 2.5 |

(continued)

| (% by mass) | | | Example | | | | | | | | | | Comparative Example | | | | | | |
|---|---|---|---|---|---|---|---|---|---|---|---|---|---|---|---|---|---|---|---|
| | | | 1 | 2 | 3 | 4 | 5 | 6 | 7 | 8 | 9 | 10 | 1 | 2 | 3 | 4 | 5 | 6 | 7 |
| Evaluation results | Dyeing properties | Black hair tress ΔE* | 8.0 | 8.3 | 11.2 | 8.6 | 5.6 | 9.3 | 7.3 | 9.4 | 8.0 | 8.0 | 12.9 | 3.2 | 3.9 | 13.5 | 9.3 | 8.2 | 8.0 |
| | | Black hair tress ΔL* | 0.7 | 3.7 | 2.8 | 2.6 | 1.1 | 2.0 | 1.1 | 1.3 | 0.7 | 0.7 | 6.5 | -1.1 | 2.5 | 5.8 | 1.7 | 0.7 | 0.7 |
| | | Bleached tress ΔE* | 18.1 | 11.5 | 23.6 | 20.4 | 12.5 | 20.7 | 20.9 | 19.2 | 18.2 | 18.1 | 9.2 | 22.6 | 9.6 | 22.0 | 20.0 | 18.4 | 18.4 |
| | | Bleached tress ΔL* | -4.5 | -2.8 | -4.4 | -8.7 | -3.5 | -5.8 | -8.0 | -6.1 | -4.3 | -4.4 | -1.1 | -12.0 | -1.4 | -11.1 | -5.9 | -4.3 | -4.2 |
| | Drying rate (min) | | 1 | 1 | 1 | 1 | 1 | 2 | 2 | 1 | 1 | 1 | 1 | 1 | 1 | 1 | 5 | 1 | 1 |
| | Smallness of color transfer after hair dyeing | | 1 | 1 | 1 | 1 | 1 | 1 | 1 | 1 | 1 | 2 | 1 | 1 | 1 | 1 | 1 | 3 | 1 |
| | Feeling on hair after hair dyeing | | 1 | 1 | 1 | 1 | 1 | 1 | 1 | 2 | 2 | 1 | 1 | 1 | 1 | 2 | 1 | 1 | 3 |

The components described in Table 1 are shown below. In addition, the blending amounts of the components described in Table 1 are each an effective amount (% by mass).

*1: Timiron Super Blue (available from Merck)
*2: Timiron Super Red (available from Merck)
*3: 70% Yellow No. 401 barium sulfate (available from Kishi Kasei Co., Ltd.)
*4: D&C RED No. 30 (available from Daito Kasei Kogyo Co., Ltd.)
*5: Blue No. 404 (color of law) (available from Kishi Kasei Co., Ltd.)
*6: OS-88E-E (available from Kao Corporation)
*7: Silicone SH 556 FLUID (available from Dow Corning Toray Co., Ltd.)

In this specification, the following Prescription Examples are disclosed.

<Prescription Example 1>

| Component | (% by mass) |
|---|---|
| Micaceous titanium A (*1) | 6.5 |
| Yellow No. 401/barium sulfate (*2) | 0.6 |
| Red No. 226 (*3) | 1.0 |
| Blue No. 404 (*4) | 1.0 |
| Trimethylsiloxysilicate (*5) | 0.5 |
| Polysilicone-9 (*6) | 7.0 |
| Methylphenyl polysiloxane (*7) | 5.0 |
| Ethanol | Balance |
| Total | 100.0 |

<Prescription Example 2>

| Component | (% by mass) |
|---|---|
| Micaceous titanium B (*8) | 4.5 |
| Yellow No. 401/barium sulfate (*2) | 1.0 |
| Red No. 226 (*3) | 1.5 |
| Blue No. 404 (*4) | 1.5 |
| Trimethylsiloxysilicate (*5) | 2.0 |
| Polysilicone-9 (*6) | 8.0 |
| Methylphenyl polysiloxane (*7) | 5.0 |
| Ethanol | Balance |
| Total | 100.0 |

The components described in the Prescription Examples are as follows.

*1: Timiron Super Blue (available from Merck)
*2: 70% Yellow No. 401 barium sulfate (available from Kishi Kasei Co., Ltd.)
*3: D&C RED No. 30 (available from Daito Kasei Kogyo Co., Ltd.)
*4: Blue No. 404 (color of law) (available from Kishi Kasei Co., Ltd.)
*5: X-21-5595 (available from Shin-Etsu Chemical Co., Ltd.)
*6: OS-88E-E (available from Kao Corporation)
*7: Silicone SH 556 FLUID (available from Dow Corning Toray Co., Ltd.)
*8: Timiron Super Red (available from Merck)

Industrial Applicability

In accordance with the present invention, it is possible to provide a hair cosmetic which is suitable as a temporary hair dye and fast in drying after application and in which color transfer to the skin or clothing after hair dyeing is less, a feeling on the hair is favorable, and color-developing behavior is hardly influenced by a base hair color, so that a fixed hair dyeing effect can be imparted.

## Claims

**1.** A hair cosmetic comprising the following components (A) to (D):

(A) an interference pearl pigment,
(B) other pigment than the component (A),
(C) a film-forming polymer, and
(D) a nonaqueous solvent,

wherein,

the total content of the component (A) and the component (B) is 2.0% by mass or more and 12.0% by mass or less,
a mass ratio [(A)/{(A) + (B)}] of the component (A) to the total content of the component (A) and the component (B) is 0.30 or more and 0.96 or less,
a mass ratio [(C)/{(A) + (B)}] of the component (C) to the total content of the component (A) and the component (B) is 0.5 or more and 2.4 or less, and
the content of water is 40% by mass or less.

**2.** The hair cosmetic according to claim 1, wherein the component (D) contains 30% by mass or more of ethanol.

**3.** The hair cosmetic according to claim 1 or 2, wherein the mass ratio [(C)/{(A) + (B)}] of the component (C) to the total content of the component (A) and the component (B) is 0.5 or more and 2.0 or less.

**4.** The hair cosmetic according to any one of claims 1 to 3, wherein the mass ratio [(C)/{(A) + (B)}] of the component (C) to the total content of the component (A) and the component (B) is 0.5 or more and 1.4 or less.

**5.** The hair cosmetic according to any one of claims 1 to 4, which is a temporary hair dye.

**6.** Use of a composition as a hair cosmetic, the composition comprising the following components (A) to (D):

(A) an interference pearl pigment,
(B) other pigment than the component (A),
(C) a film-forming polymer, and
(D) a nonaqueous solvent,

wherein,

the total content of the component (A) and the component (B) is 2.0% by mass or more and 12.0% by mass or less,
a mass ratio [(A)/{(A) + (B)}] of the component (A) to the total content of the component (A) and the component (B) is 0.30 or more and 0.96 or less,
a mass ratio [(C)/{(A) + (B)}] of the component (C) to the total content of the component (A) and the component (B) is 0.5 or more and 2.4 or less, and
the content of water is 40% by mass or less.

## INTERNATIONAL SEARCH REPORT

International application No.
PCT/JP2020/015484

A. CLASSIFICATION OF SUBJECT MATTER
Int.Cl. A61K8/898(2006.01)i, A61K8/19(2006.01)i, A61K8/25(2006.01)i, A61K8/29(2006.01)i, A61K8/34(2006.01)i, A61K8/42(2006.01)i, A61K8/49(2006.01)i, A61K8/58(2006.01)i, A61K8/89(2006.01)i, A61Q5/00(2006.01)i, A61Q5/06(2006.01)i
FI: A61K8/898, A61K8/19, A61K8/25, A61K8/29, A61K8/34, A61K8/42, A61K8/49, A61K8/58, A61K8/89, A61Q5/00, A61Q5/06
According to International Patent Classification (IPC) or to both national classification and IPC

B. FIELDS SEARCHED
Minimum documentation searched (classification system followed by classification symbols)
Int.Cl. A61K8/898, A61K8/19, A61K8/25, A61K8/29, A61K8/34, A61K8/42, A61K8/49, A61K8/58, A61K8/89, A61Q5/00, A61Q5/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2005-239626 A (KANEBO COSMETICS INC.) 08.09.2005 (2005-09-08), claims, paragraphs [0001], [0013]-[0017], [0036], table 1 | 1-6 |
| Y | JP 2001-199850 A (KANEBO LTD.) 24.07.2001 (2001-07-24), paragraph [0022] | 1-6 |
| X | JP 2007-308393 A (HOYU CO., LTD.) 29.11.2007 (2007-11-29), claims, paragraphs [0008], [0011], table 1 | 1-6 |
| A | JP 11-12135 A (KAO CORPORATION) 19.01.1999 (1999-01-19), claims | 1-6 |
| A | JP 2017-114815 A (KAO CORPORATION) 29.06.2017 (2017-06-29), claims | 1-6 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09.06.2020 | 16.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/015484

| | | |
|---|---|---|
| JP 2005-239626 A | 08.09.2005 | (Family: none) |
| JP 2001-199850 A | 24.07.2001 | (Family: none) |
| JP 2007-308393 A | 29.11.2007 | (Family: none) |
| JP 11-12135 A | 19.01.1999 | EP 887067 A2<br>claims<br>US 6190648 B1 |
| JP 2017-114815 A | 29.06.2017 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017114815 A
- JP 2019006690 A
- JP 2019006691 A